# EUROPEAN PATENT APPLICATION

(11) **EP 1 860 665 A1**
(43) Date of publication of application: **28.11.2007**
(21) Application number: 06425345.3
(22) Date of filing: 23.05.2006
(51) Int. Cl.: G21F 7/06, A61M 5/178, B65B 3/00, A61J 1/00

(54) **Double needle element for dispensing radiofluids**

(71) Applicant: Comecer S.p.A., 48014 Castel Bolognese (IT)
(72) Inventor: Bedeschi, Paolo, 48014 Castel Bolognese (IT)
(74) Representative: Jorio, Paolo

(57) **Abstract**

A double needle element (1) for dispensing radiofluids, consists of a supporting body (2), in which at a first end (2a) it is made a connection cavity (4) adapted to accommodate part of a container to be filled, and at a second end (2b) there are made two cylindrical cavities (5) each of which is adapted to accommodate an end of a respective tube; the double needle element comprises a pair of needles (7) fixedly accommodated within the supporting body (2), each of which has an output end portion (8) accommodated within the connection cavity (4), and an input end (9) connected to a respective cylindrical cavity (5).

## Description

The present invention relates to a double needle element for dispensing radiofluids.

In machines for the preparation of radiofluid-based products, such as for example radiopharmaceuticals, the dispensing operation envisages the use of two separate conduits, each of which ends with a dripping needle within the container to be filled. The two tubes lead respectively to a dilution solution, e.g. physiological solution, and to a concentrated radioisotope solution. The presence of dripping needles derives from the need for ensuring a very slow filling, in order to be able to control radioactive activity by volume with the maximum accuracy.

Generally, the container to be filled is closed with a rubber cap, which during the filling operation is fluid-tightly crossed by the dripping needles. Once the dispensing step is completed and the container is appropriately filled, the operator removes the two needles separately one with respect to the other.

Such a procedure, in which the two needles are handled separately one with respect to the other and without any form of support, causes considerably risks for the operator, in particular considering that such needles come into contact with radioactive products.

It is the object of the present invention to make a device whose technical features ensure the absence of risks for the operator, while increasing practicality of the dispensing operation at the same time.

The object of the present invention is a double needle element for dispensing radiofluids, comprising a supporting body presenting a connection cavity adapted to accommodate part of a container to be filled, and two cylindrical cavities each of which is adapted to accommodate an end of a respective tube; said double needle element comprising a pair of needles fixedly accommodated within said supporting body, and each of which has an output end portion accommodated within said connection cavity and an input end connected to a respective cylindrical cavity.

The following example is provided by way of nonlimiting illustration for better understanding of the invention with the help of the figures in the accompanying drawing, in which:
- figure 1 is a cross-section of a double needle element according to the present invention; and
- figure 2 is a cross-section of the element in figure 1, coupled to other parts of a machine for dispensing radioactive products.

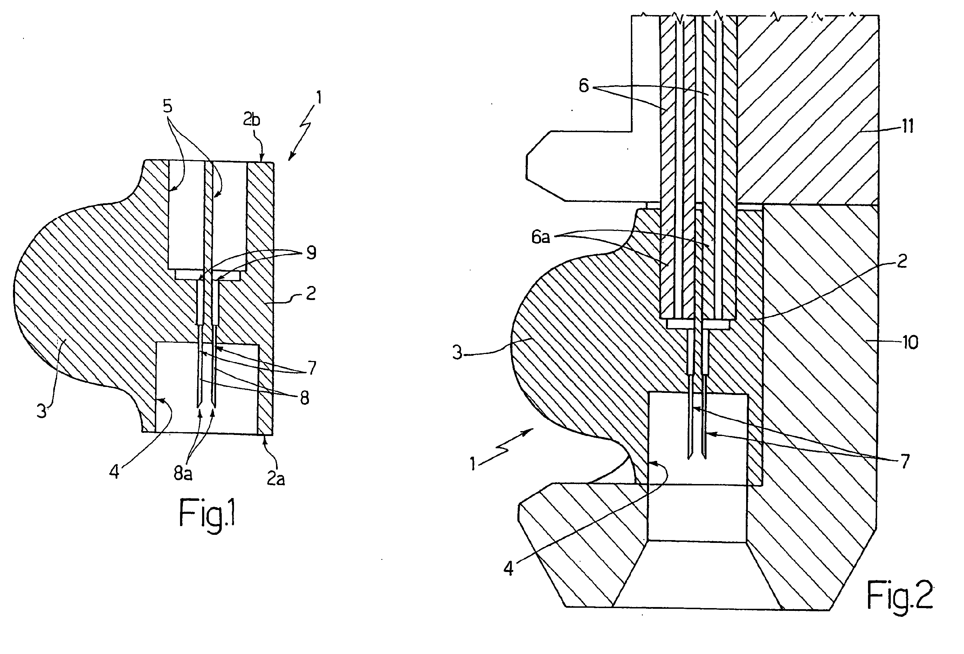

In figure 1, it is indicated as a whole by 1 a double needle element object of the present invention.

Element 1 comprises a supporting body 2 presenting a cylindrical shape from whose external surface a gripping tongue 3 protrudes. A connection cavity 4 is made in supporting body 2 facing a first circular end wall 2a, and is adapted to accommodate an end of the container to be filled with the radioactive product, such as for example a disposable syringe.

Two reciprocally parallel cylindrical cavities 5 are made in supporting body 2, each of which faces a second circular end wall 2b. As shown in figure 2, each of the cylindrical cavities 5 is adapted to accommodate one end a of a respective fluid conveying tube 6 as shown in figure 2. In particular, one of the cylindrical cavities 5 accommodates tube 6 conveying the dilution fluid, while the other accommodates tube 6 conveying the concentrated radioisotope solution.

Element 1 also comprises two needles 7 fixedly accommodated within supporting body 2. Each of the needles 7 presents a portion 8 comprising a release end 8a accommodated within the connection cavity 4, and a respective inlet 9 which is connected to a respective cylindrical cavity 5 so as to directly receive the fluid conveyed by the respective tube 6 accommodated in the cylindrical cavity itself.

As shown in figure 2, element 1 object of the present invention is inserted in a specific support 10 fixed to an elevator arm 11 of the machine.

Ends 6a of the two tubes 6 conveying the solutions are accommodated in the two respective cylindrical cavities 5 and glued.

In use, double needle element 1 is inserted in support 10. At this point, a disposal syringe is arranged with an end accommodated within the connection cavity 4 so that the release ends 8a of needles 7 draw from within the syringe itself. Usually, the end of the syringe is closed with a rubber cap, which during positioning of the connection cavity 4 is perforated by two needles 7 which make the two release ends 8a draw from inside the syringe.

Each of needles 7 receives fluid from respective tube 6 and drips it within the syringe.

Once the dispensing operation is completed and the syringe is appropriately filled, connection cavity 4 is disengaged from the syringe itself, and double needle element 1 is removed conveniently and in complete safety from support 10, along with the two tubes 6.

If the container to be filled is a flask instead of a syringe, the inlet of the flask is taken underneath connection cavity 4 and the product is dripped into the flask itself. Once filled, the flask is closed with a rubber cap and fixed with an aluminium ring.

As it is apparent in the above description, the double needle element according to the present invention ensures both that the operator cannot be injured by the needle tips, because they are protected by the supporting body, and makes connection and disconnection operations of the tubes and of the respective needles for the dispensing operation extremely simple.

## Claims

1. A double needle element (1) for dispensing radiofluids, comprising a supporting body (2) presenting a connection cavity (4) adapted to accommodate part of a container to be filled, and two cylindrical cavities (5) each of which is adapted to accommodate an end of a respective tube; said double needle element comprising a pair of needles (7) fixedly accommodated within said supporting body (2), and each of which has an output end portion (8) accommodated within said union cavity (4) and an input end (9) connected to a respective cylindrical cavity (5).

2. A double needle element according to claim 1, **characterised in that** it comprises a supporting body (2) having cylindrical shape from whose external surface a gripping tongue (3) extends.

3. A double needle element according to claim 2, **characterised in that** said connection cavity (4) faces a first circular end wall (2a) of said supporting body (2), and **in that** said pair of cylindrical cavities (5) face a second circular end wall (2b) of said supporting body (2) .
